# EUROPEAN PATENT SPECIFICATION

(11) **EP 4 440 525 B1**
(45) Date of publication and mention of the grant of the patent: **11.03.2026**
(21) Application number: 22823523.0
(22) Date of filing: 30.11.2022
(51) Int. Cl.: A61F 13/38, B65D 65/42, D21H 17/24, D21H 17/28, D21H 19/34, D21H 27/10

(54) **HEAT-SEALABLE PAPER**
HEISSSIEGELBARES PAPIER
PAPIER THERMOSCELLABLE

(30) Priority: 03.12.2021 EP 21212318
(43) Date of publication of application: 09.10.2024
(73) Proprietor: Billerud Aktiebolag (publ), 169 27 Solna (SE)
(72) Inventor: STÖÖD, Mikael, 654 64 Karlstad (SE); SAARI, Markus, 20900 Turku (FI)
(74) Representative: Kransell & Wennborg KB
(86) International application number: PCT/EP2022/083955
(87) International publication number: WO 2023/099625

(56) References cited:
- WO-A1-2018/116118
- JP-A- H1 085 679
- US-A- 3 071 485

## Description

### TECHNICAL FIELD

The present disclosure relates to the field of heat-sealable papers and in particular to recyclable heat-sealable papers.

### BACKGROUND

Heat-sealable materials are used in various packaging applications. Traditionally, the packaging material has been a free-standing plastic material, such as a film. Alternatively, a plastic material has been coated onto a non-sealable material, such as paper or board, and sealed together with a free-standing plastic material, such as a film. The sealed package is protecting its content from the outside environment.

### SUMMARY

The present disclosure aims to provide a recyclable plastic-free heat-sealable paper-based material that can replace plastic materials in packaging applications.

The invention is defined by the claims. Furthermore, the present disclosure provides the following listing of itemized embodiments:
1. Method of forming a package or container or paper stick comprising the steps of:
   a) providing a paper product comprising a paper substrate and a film coated on the paper substrate, wherein the film comprises oxidized starch in an amount of 5-95 %, such as 10-50 %, such as 10-30 %, based on the dry weight of the film and dextrin, such as maltodextrin in an amount of 95-5 %, such as 90-50 %, such as 90-70 %, based on the dry weight of the film;;
   b) wetting a portion of the film; and
   c) heat-sealing the wetted portion.
2. The method of item 1, wherein the paper product is sealed to itself in step c).
3. The method of item 1, wherein the paper product is sealed to a second paper product in step c).
4. The method of item 3, wherein the second paper product comprises a second paper substrate and a second film coated on the second paper substrate, wherein the second film comprises oxidized starch and dextrin, such as maltodextrin.
5. The method of any one of items 1-4, wherein two wetted films comprising oxidized starch and dextrin, such as maltodextrin, are contacted with each other in step c).
6. The method of any one of item 1-5, wherein the film further comprises a wet-strength agent.
7. The method of item 6, wherein the wet-strength agent is present in an amount of 3-7 %, such as 4-6 %, based on the dry weight of the film.
8. The method of any of the preceding items, wherein the grammage of the film is 0.5-10 g/m², such as 0.5-5 g/m², such as 0.5-3 g/m², such as 1-3 g/m².
9. The method of any of the preceding items wherein the film comprises at least 50 %, such as at least 70 %, such as at least 90 % oxidized starch and dextrin based on the dry weight of the film
10. The method of any of the preceding items, wherein the moisture content of the film in step a) is less than 30%, such as less than 20%, such as less than 10%, based on the dry weight of the film.
11. The method of any one of the preceding items, wherein the paper substrate is a paper the density measured according to ISO 534:2011 of the paper substrate is 700-900 kg/m³, such as 750-900 kg/m³, such as 800-900 kg/m³.
12. The method of any one of the preceding items, wherein the paper substrate is a paper the grammage measured according to ISO 536:2020 of the paper substrate is 30-90 g/m², such as 35-55 g/m², such as 55-85 g/m².
13. The method of any one of the preceding items, wherein the paper substrate is a paper the grammage measured according to ISO 536:2020 of the paper product is 32-92 g/m², such as 37-57 g/m², such as 57-87 g/m².
14. The method of any of the preceding items, wherein the paper substrate is a machine-glazed (MG) paper having a smooth side and a rough side.
15. The method of item 14 wherein the film is coated on the smooth side of the paper substrate.
16. The method of item 15, wherein the grammage of the film is 0.5-1.5 g/m².
17. The method of item 14, wherein the film is coated on the rough side of the paper substrate.
18. The method of item 17, wherein the grammage of the film is 1-3 g/m², such as 1-2 g/m2.
19. The method of any of the items 1-10, wherein the method is a method for producing a package or container and the paper substrate is a paperboard.
20. The method of item 19, wherein the density measured according to ISO 534:2011 of the paper substrate is 550-900 kg/m³, such as 550-750 kg/m³.
21. The method of item 19 or 20, wherein the grammage measured according to ISO 536:2020 of the paper substrate is 150-400 g/m², such as 200-350 g/m².
22. The method of any of items 19-21, wherein the grammage measured according to ISO 536:2020 of the paper product is 152-402 g/m², such as 202-352 g/m².
23. The method of any of the preceding items wherein the heat sealing is performed at a temperature in the interval of 110 °C - 200 °C, such as 110 °C - 190 °C, such as such as 150 °C - 188 °C.
24. The method of any of the preceding items wherein the heat sealing is performed at a pressure of 3-10 bar.
25. The method of any of the preceding items wherein the heat sealing is performed at a press time of 1-10 seconds.
26. The method of any of one of items 1-18, wherein the method is a method of forming a paper stick further comprising the step:
   b1) rolling the paper into a paper stick, wherein step b1) is conducted after step a) and before step c).
27. The method of item 26, wherein step b) and step b1) are conducted in a paper stick production machine.
28. The method of item 27 ,wherein step b) is conducted during step b1) in the paper stick production machine.
29. The method of any one of items 26-28, wherein the portion of the film being wetted is forming at least part of an outer layer in the paper stick, such as forming part of the two outermost layers in the paper stick.
30. The method of any one of items 26-29, wherein the drying is non-contact drying, such as IR-drying and/or hot air drying.
31. The method of any one of items 26-30, wherein the paper substrate is a machine-finished (MF) or machine-glazed (MG) paper.
32. The method of item 31, wherein the grammage measured according to ISO 536:2020 of the paper substrate is 50-90 g/m², such as 55-85 g/m².
33. A paper stick produced according to any one of the items 26-32.
34. A cotton bud comprising the paper stick produced according to any one of the items 26-32.
35. A sealed package or container comprising a paper product comprising a paper substrate and a film coated on the paper substrate, wherein the film comprises oxidized starch and dextrin, such as maltodextrin.
36. The package or container of item 35, wherein the paper product has been sealed to itself.
37. The package or container of item 35, wherein the paper product has been sealed to a second paper product.
38. The package or container of item 37, wherein the second paper product comprises a second paper substrate and a second film coated on the second paper substrate, wherein the second film comprises oxidized starch and dextrin, such as maltodextrin.
39. The package or container of any one of items 35-38, wherein the package or container comprises a sealing line and a product within the package or container, wherein the sealing line is arranged so that a product is sealed within the package or container.
40. The package or container of any one of items 35-38, wherein the package or container comprises a sealing arranged so that the package or container is partly open so that a product can be inserted and removed without breaking the sealing line or the package or container.

Oxidized starch and dextrin are combined in one coating. Such combination provides a synergistic effect. Oxidized starch as well as dextrin have long been known to be used individually as adhesives for paper. However, the inventors have now realized that when combined into one single coating, a heat-sealable coating is provided that forms a superficial film being absorbed just enough by the paper substrate upon coating to provide for heat-sealability by wetting the film without the need for a plastic heat-sealable film.

### BRIEF DESCRIPTION OF THE DRAWINGS

Aspects and embodiments are now described, by way of example, with reference to the accompanying drawings, in which:
Fig 1 shows a schematic image of an exemplary embodiment of an open package or container comprising a sealing arranged so that the sealing line partly surrounds a product within the package or container.
Fig 2 shows a schematic image of another exemplary embodiment of an open package or container comprising a sealing arranged so that the sealing line partly surrounds a product within the package or container.
Fig 3 shows a schematic image of an exemplary embodiment of a closed package or container comprising a sealing line surrounding a product within the package or container.
Fig 4 shows a schematic image of a paper stick.
Fig 5 shows a schematic image of a cotton bud comprising a paper stick.

### DETAILED DESCRIPTION

As a first aspect of the present disclosure, there is provided a method of forming a package or container or paper stick comprising the steps of:
a) providing a paper product comprising a paper substrate and a film coated on the paper substrate, wherein the film comprises oxidized starch in an amount of 5-95 %, such as 10-50 %, such as 10-30 %, based on the dry weight of the film and dextrin, such as maltodextrin, in an amount of 95-5 %, such as 90-50 %, such as 90-70 %, based on the dry weight of the film;;
b) wetting a portion of the film; and
c) heat-sealing the wetted portion.

The oxidized starch and dextrin in the film is sufficient for providing the heat-sealability without the use of any additional polymer layers. Moreover, if the paper substrate is not coated with any plastic film, the package or container or paper stick produced is, thus, bio-based as well as the need for plastics is reduced or even avoided. A package or container of such type is typically a grease-proof package such as an open bag that can be used for example for french fries or pastry goods or a sealed bag that can be used for example for cereals or porridge or a sealed paperboard package or container.

The inventors have realized that by using a combination of oxidized starch and dextrin in the coating, it is possible to coat a paper substrate with a film that is absorbed enough to stick to the paper substrate but not too much to be absorbed into, and impregnate, the paper substrate. Thereby a superficial film can be formed. Wetting of the film is then sufficient to provide heat-sealability. Preferably, 10-50 % by dry weight of the film is oxidized starch and 90-50 % by dry weight of the film is dextrin. More preferably, 10-30 % by dry weight of the film is oxidized starch and 90-70 % by dry weight of the film is dextrin. Besides synergistically providing a heat-sealing capability, the dextrin is believed to provide barrier properties while the oxidized starch is believed to provide a film forming capability.

The paper product may be sealed to itself in step c). In such case, a package or bag or paper stick can be produced from a single paper substrate by folding or rolling the paper product followed by heat-sealing. Alternatively, the paper product is sealed to a second paper product in step c). In such case, a package or bag can be produced without the need for folding any of the paper products. Moreover, production of a pouch being of different shapes on top and bottom side is facilitated. The second paper product is in such case typically comprising a paper substrate being coated with a film, wherein the film comprises oxidized starch and dextrin, such as maltodextrin.

The paper substrate is typically a paper or a paperboard.

Typically, two wetted films comprising oxidized starch and dextrin, such as maltodextrin, are contacted with each other in step c).

In one embodiment, the film further comprises a wet-strength agent. In such embodiment, the wet-strength agent is typically present in an amount of 3-7 %, such as 4-6 %, based on the dry weight of the film. Addition of a wet strength agent is beneficial for the strength of the portion of the film being wetted.

The grammage of the film is typically 0.5-10 g/m², such as 0.5-5 g/m², such as 0.5-3 g/m², such as 1-3 g/m². A too low grammage can impair the heat-seal and a too high grammage increases the cost.

The film typically comprises at least 50 %, such as at least 70 %, such as at least 90 % oxidized starch and dextrin based on the dry weight of the film.

The moisture content of the film in step a) is typically less than 30%, such as less than 20%, such as less than 10%, based on the dry weight of the film. The film-coated paper substrate is typically, at least partially, dried prior to heat-sealing. The coated paper substrate may therefore be produced at one site, followed by drying and transportation to another site where it is heat-sealed into a bag or package or paper stick. Alternatively, it is dried and heat-sealed into a bag or package or paper stick at the same facility.

In embodiments the paper product may be a package or container and the paper substrate a paper. The grammage measured according to ISO 536:2020 of the paper substrate is typically 30-60 g/m², such as 35-55 g/m². A suitable density (measured according to ISO 534:2011) for the paper substrate is 700-900 kg/m³, such as 750-900 kg/m³, such as 800-900 kg/m³. Typically for a package or container where the paper substrate is a paper, a too low density is not suitable since such paper is too porous for coating with the film comprising oxidized starch and dextrin. When forming forming a package or container, the grammage measured according to ISO 536:2020 of the paper product is typically 32-62 g/m², such as 37-57 g/m².

The paper substrate may be a machine-glazed (MG) paper or a machine-finished (MF) paper. The MG paper is having a smooth side and a rough side. The MG paper or MF paper may be calendered. The MG paper or MF paper is typically a kraft paper, and typically at least 80%, preferably at least 90%, by dry weight of the fibres used to produce the MG paper are virgin fibres. The smooth side of the MG paper is the side that faced the Yankee cylinder (a polished metal cylinder sometimes referred to as a MG cylinder) used for drying the paper web in the MG papermaking machine. The contact with the polished metal surface during drying makes the smooth side smoother than the rough side. MF paper is instead dried by a large number of smaller, steam-heated cylinders to dry the paper which is alternately wrapped one way and then the other way so that both sides of the paper receive the same finish. The finish on both sides of an MF paper is typically similar to the rough side of an MG paper.

Typically, when the paper substrate is a MG paper, the film is coated on the smooth side of the paper substrate. Coating on the smooth side is especially beneficial if a package is produced in a Multivac sealing machine. In such case the grammage of the film is advantageously 0.5-2.5 g/m², such as 0.5-1.5 g/m². A Multivac sealing machine is a widely used sealing equipment for packaging of foodstuffs in sealed packages. A common use of the Multivac sealing equipment is to arrange a sheet or a tray, typically made of plastic or plastic-coated paper, with foodstuff on top and an additional sheet arranged on top of the food followed by heat-sealing to enclose the foodstuff inside a sealed package. Thereby, a package or container can be formed consisting of a paper tray and a substantially flat paper on top. Alternatively, a pouch having similar shape on both papers can be produced.

Alternatively, the film is coated on the rough side of the MG paper substrate and in such case the grammage of the film is typically 1-3 g/m², such as 1-2 g/m².

The heat-sealing in step c) is typically performed at a temperature in the interval of 110 °C - 200 °C, such as 110 °C - 190 °C, such as such as 150 °C - 188 °C. The heat-sealing in step c) is typically performed at a pressure of 3-10 bar. The heat-sealing in step c) is typically performed at a press time of 1-10 seconds.

The film has typically been formed from a coating mixture applied by means of a film press or a size press. Alternatively, the coating mixture has been applied by spraying. In yet another alternative, a curtain coater, direct rod coater or blade coater has been used to apply the coating mixture forming the film.

In the embodiment where the method is a method of forming a paper stick, the method further comprising the step:
b1) rolling the paper into a paper stick, wherein step b1) is conducted after step a) and before step c).

Typically step b) and step b1) are conducted in a paper stick production machine, and preferably step b) is conducted during step b1) in the paper stick production machine. By conducting step b) during step b1) the efficiency is increased since the same machine can be used for wetting and rolling.

Typically, the portion of the film being wetted is forming at least part of an outer layer in the paper stick, such as forming part of the two outermost layers in the paper stick. It is preferred that at least a portion of the outer layer has been wetted as it otherwise will be a loose edge. Heat-sealing in step c) is typically conducted by drying the paper stick with the outer layer being wetted to seal the rolled paper stick. Drying is typically a non-contact drying, such as IR-drying and/or hot air drying. The paper substrate is typically a machine-finished (MF) or machine-glazed (MG) paper and the grammage of the paper substrate is as measured according to ISO 536:2020 is typically 50-90 g/m², such as 55-85 g/m², and the grammage measured according to ISO 536:2020 of the paper product is typically 52-92 g/m², such as 57-87 g/m².

In embodiments where the paper product may be a package or container and the paper substrate a paperboard. The density measured according to ISO 534:2011 of the paper substrate is typically 550-900 kg/m³, such as 550-750 kg/m³. The grammage measured according to ISO 536:2020 of the paper substrate is typically 150-400 g/m², such as 200-350 g/m². The grammage measured according to ISO 536:2020 of the paper product is typically 152-402 g/m², such as 202-352 g/m².

As a second aspect of the present disclosure, there is a paper stick produced according to the first aspect of the present disclosure. There is also disclosed a cotton bud comprising the paper stick produced according to the first aspect of the present disclosure. Cotton buds consist of one or two small wads of cotton wrapped around one or both ends of a short rod made of paper stick. They may typically be used for ear cleaning, first aid, cosmetics application, cleaning, and crafts.

As a third aspect of the present disclosure, there is provided a sealed package or container comprising a paper substrate and a film coated on the paper substrate, wherein the film comprises oxidized starch and dextrin, such as maltodextrin.

The paper product may have been sealed to itself to produce the package or container. Alternatively, the paper product may have been sealed to a second paper product to produce the package or container. Typically, the second paper product comprises a film, wherein the film comprises oxidized starch and dextrin, such as maltodextrin.

In one embodiment, the package or container comprises a sealing line and a product within the package or container, wherein the sealing line is arranged so that a product is sealed within the package or container. In such embodiment, the product is enclosed inside the sealed package independently on which direction the package is arranged in. Example of such package is a closed sealed pouch sealed all around the product. Such product may be produced using a Multivac-equipment or similar. Alternatively, the package is folded around the product and the open edges being sealed.

In an alternative embodiment, the package or container comprises a sealing arranged so that the package or container is partly open so that a product can be inserted and removed without breaking the sealing line or the package or container. Example of such package is a bag sealed on all sides but one leaving an opening, or a folded paper sealed on one edge leaving an opening.

The examples and embodiments discussed above in connection to the first aspect apply to the second and third aspects *mutatis mutandis.*

The present disclosure will now be described hereinafter with reference to the accompanying drawings, in which certain embodiments of the invention are shown.

These aspects may, however, be embodied in many different forms and should not be construed as limiting; rather, these embodiments are provided by way of example so that this disclosure will be thorough and complete, and to fully convey the scope of all aspects of invention to those skilled in the art. Like numbers refer to like elements throughout the description.

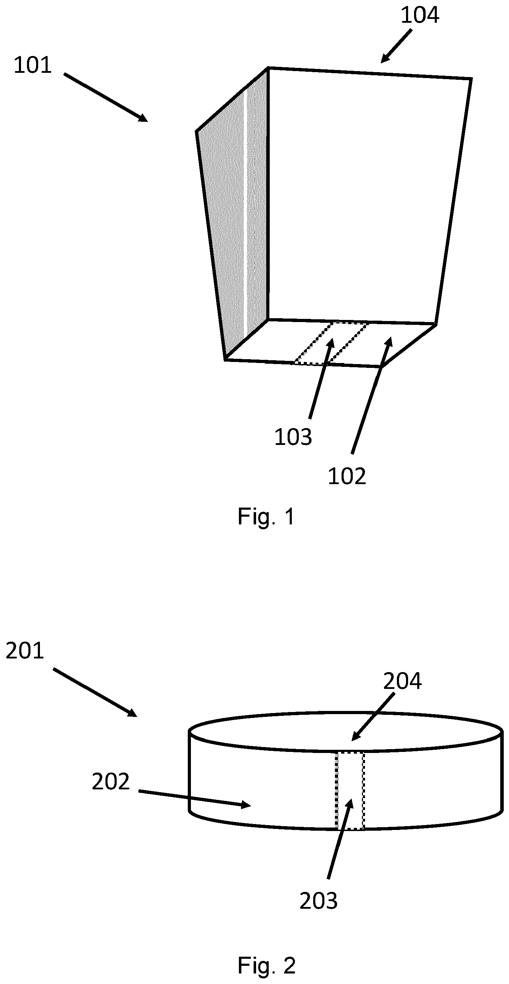

Fig 1 illustrates a schematic image from a bottom-side perspective of a package 101 having a bottom 102 sealed by a sealing line 103 from a film comprising oxidized starch and dextrin and being open on the top 104.

Fig 2 illustrates a schematic image from a top-side perspective of a package 201 having a side 202 sealed by a sealing line 203 from a film comprising oxidized starch and dextrin and being open on the top 204.

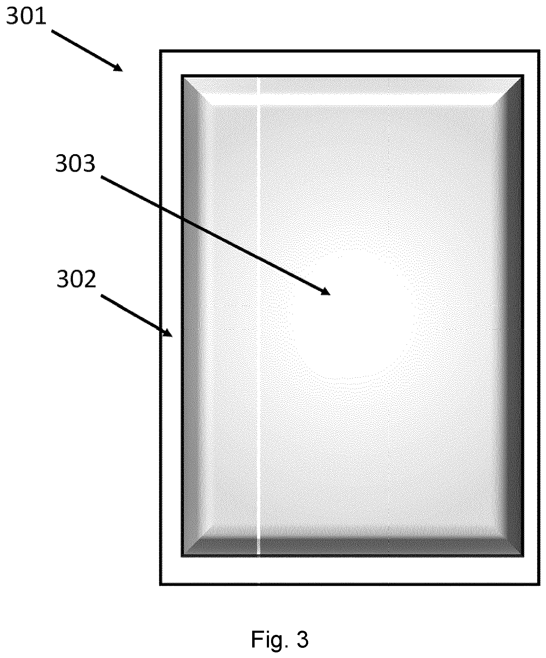

Fig 3 illustrates a schematic image from a top perspective of a closed package 301 sealed around a product 303 by a sealing line 302 from a film comprising oxidized starch and dextrin.

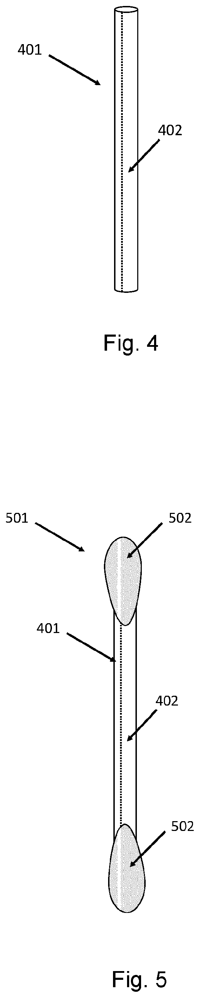

Fig 4 illustrates a schematic image of a rolled paper stick 401 having a sealing line 402 illustrating the outer layer being sealed to the second outermost layer.

Fig 5 illustrates a schematic image of a cotton bud 501 comprising a rolled paper stick 401 having a sealing line 402 illustrating the outer layer being sealed to the second outermost layer as well as two wads of cotton 502 wrapped around both ends of the paper stick 401.

### EXAMPLES

### Coating of paper

Machine glazed (MG) paper (40 g/m²) was coated by the use of a film press with a coating consisting of Perfectafilm B4085 from Avebe (100 pph) and Kymene GHP20 from Solenis (5 pph). Perfectafilm B4085 is a mixture of oxidized starch (20 wt%) and maltodextrin (80 wt%). The MG paper was coated either on the smooth side (MG-OX/MD) with a grammage of 1 g/m² or on the rough side (RS-OX/MD) with a grammage of about 1.5 g/m².

Moreover, in addition to the mixture in the product Perfectafilm B4085, different mixtures of oxidized starch and maltodextrin were prepared. In addition, two reference coatings being either free of oxidized starch or free of maltodextrin were prepared. These were all coated on an MG paper with a grammage of about 2 g/m². The coatings were coated on the smooth side of the MG paper apart from the reference free of maltodextrin (RS-100/0) that was coated on the rough side. These mixtures and references are presented in Table 1.

**Table 1. Manually prepared mixtures of oxidized starch and maltodextrin and coated at a coat weight of about 2 g/m².**

| Sample | Oxidized starch (wt%) | Maltodextrin (wt%) |
|---|---|---|
| MG-0/100 | 0 | 100 |
| MG-10/90 | 10 | 90 |
| MG-20/80 | 20 | 80 |
| MG-40/60 | 40 | 60 |
| MG-80/20 | 80 | 20 |
| RS-100/0 | 100 | 0 |

Three additional references were prepared: MG paper coated on the MG side with a polyethylene coating (MG-PE), MG paper coated on the MG side with a maltodextrin coating (MG-MD), free of oxidized starch, with a grammage of 1 g/m², and MG paper without any coating (non-coated) (MG).

### Seal test

The coated sides of two MG-OX/MD papers were wetted with water, put together and sealed under heat (180 °C, 195 °C or 200 °C) and pressure (7 bar) for 5 seconds (at 180°C), 9 seconds (at 195°C) or 3 seconds (at 200 °C) to form sealed strips. The coating in these papers were made with Perfectafilm B4085 and accordingly contained 20 wt% oxidized starch and 80 wt% maltodextrin.

The manually prepared mixtures of oxidized starch and maltodextrin were wetted with water, put together and sealed under heat (180 °C) and pressure (7 bar) for 5 seconds.

The coated sides of two MG-MD papers were wetted with water, put together and sealed under heat (180°C) and pressure (7 bar) for 5 seconds. Two MG papers (non-coated) were also sealed under heat (180°C) and pressure (7 bar) for 5 seconds.

The coated sides of two MG-PE papers were put together and sealed under heat (170 °C) and pressure (4 bar) for 4 seconds.

Maximum heat seal strength (N) was thereafter evaluated according to ASTM F88 & EN 868-5 on a 15 mm test strip and the results are presented in Table 2.

**Table 2. The seal strength of sealed papers.**

| **Sealed papers** | **Seal strength (N/15mm)** |
|---|---|
| MG-OX/MD (180°C) | 1.1 |
| MG-OX/MD (195°C) | 0.7 |
| MG-OX/MD (200°C) | 0.6 |
| MG-PE (170 °C) | 1.2 |
| MG-MD (180 °C) | 0.4 |
| MG (180 °C) | * |
| MG-0/100 | 0.4 |
| MG-10/90 | 1.9 |
| MG-20/80 | 1.8 |
| MG-40/60 | 1.9 |
| MG-80/20 | 1.5 |
| RS-100/0 | 0.1 |

| | |
|---|---|
| *sticks somewhat together after heating so value above 0 N/15mm, but estimated to be below 0.4 N/15mm | |

As shown below in the Multivac test, heat-sealability can be achieved on both sides (MG and RS) of the paper. Hence it is concluded that the unfavourable seal strength value obtained for RS-100/0 in table 2 above is not primarily an effect of the side of the paper that was coated, but of the composition of the coating. Accordingly, it is concluded that dextrin must be included in the coating to achieve sufficient seal strength.

The reason why the manually prepared mixture of MG-20/80 obtained a higher seal strength than coating with Perfectafilm B4085 containing 20 wt% oxidized starch and 80 wt% maltodextrin is that the coating grammage was higher for the manually prepared mixture, 2 g/m² instead of 1 g/m². For the reference, MG-MD (180 °C) and MG-0/100, on the other hand, they were also coated at different grammages, but there the seal strength did not improve with increased coating grammage.

### Multivac test

In a Multivac machine regularly used for sealing a plastic film onto a tray, a sealing test was performed. The coated sides of two MG-OX/MD papers were put together and sealed under heat (110 °C) and pressure (7 bar) for 1.5 seconds to form a sealed pouch. In the same way 2 more pouches were prepared from the following: one RS-OX/MD paper and one MG-OX/MD paper as well as two RS-OX/MD papers.

Seal strength was evaluated for the pouch prepared from two MG-OX/MD papers. Moreover, two sets of all 3 pouches were prepared and the first set was tested for fiber tear when intending to separate the two papers in each pouch and the second set was kept for long-term testing by visual inspection of the seal after 3 months.

| **Sealed papers** | **Seal strength (N/15mm)** | **Fiber tear?** |
|---|---|---|
| MG-OX/MD + MG-OX/MD | 1.1 | Yes |
| MG-OX/MD + RS-OX/MD | * | Yes |
| RS-OX/MD + RS-OX/MD | * | Yes |

| | | |
|---|---|---|
| *Not tested | | |

For the first set of pouches, all of them displayed fiber tear when pulling apart the papers. The appearance of fiber tear shows that the seal is stronger than the papers themselves so that a coherent failure occurs, i.e. a strong seal had been formed independently on which side of the paper that had been coated.

For the second set of pouches, still 3 months after sealing, the seal between the papers in all 3 pouches was intact. This further shows that coating on both MG and RS side provides a strong seal.

The experimental data shows that a coating based on oxidized starch and dextrin provides for a seal strength both as free-standing strips and when produced as pouches in the Multivac that is well-comparable to that of a sealed PE-coating.

Moreover, the data also shows that it is the combination of oxidized starch and dextrin that provides this effect as paper coated with only maltodextrin or only oxidized starch as well as paper without coating does not yield the same seal strengths. All tested mixtures of oxidized starch and dextrin (ranging from 80/20 to 10/90) resulted in a seal strength comparable to that obtained with a PE-coated paper.

## Claims

1. Method of forming a package or container or paper stick comprising the steps of:
a) providing a paper product comprising a paper substrate and a film coated on the paper substrate, wherein the film comprises oxidized starch in an amount of 5-95 %, such as 10-50 %, such as 10-30 %, based on the dry weight of the film and dextrin, such as maltodextrin, in an amount of 95-5 %, such as 90-50 %, such as 90-70 %, based on the dry weight of the film;
b) wetting a portion of the film; and
c) heat-sealing the wetted portion.

2. The method of claim 1, wherein the paper product is sealed to itself in step c).

3. The method of claim 1, wherein the paper product is sealed to a second paper product in step c).

4. The method of claim 3, wherein the second paper product comprises a second paper substrate and a second film coated on the second paper substrate, wherein the second film comprises oxidized starch and dextrin, such as maltodextrin.

5. The method of any one of claims 1-4, wherein the film further comprises a wet-strength agent.

6. The method of claim 5, wherein the wet-strength agent is present in an amount of 3-7 %, such as 4-6 %, based on the dry weight of the film.

7. The method of any of the preceding claims, wherein the grammage of the film is 0.5-5 g/m², such as 0.5-3 g/m², such as 1-3 g/m².

8. The method of any of the preceding claims wherein the film comprises at least 50 %, such as at least 70 %, such as at least 90 % oxidized starch and dextrin based on the dry weight of the film.

9. The method of any of the preceding claims, wherein the paper substrate is a machine-glazed (MG) paper having a smooth side and a rough side and the film is coated on the smooth side or the rough side of the paper substrate.

10. The method of any of any of the preceding claims, wherein the method is a method of forming a paper stick further comprising the step:
b1) rolling the paper into a paper stick, wherein step b1) is conducted after step a) and before step c).

11. A paper stick produced according to claim 10.

12. A cotton bud comprising the paper stick produced according to claim 10.

13. A sealed package or container comprising a paper product comprising a paper substrate and a film coated on the paper substrate, wherein the film comprises oxidized starch and dextrin, such as maltodextrin.

14. The package or container of claim 13, wherein the package or container comprises a sealing line and a product within the package or container, wherein the sealing line is arranged so that a product is sealed within the package or container.

15. The package or container of claim 13, wherein the package or container comprises a sealing arranged so that the package or container is partly open so that a product can be inserted and removed without breaking the sealing line or the package or container.

## Patentansprüche

1. Verfahren zur Bildung einer Verpackung oder eines Behälters oder eines Papierstabs, aufweisend die Schritte:
a) Bereitstellen eines Papierprodukts, das ein Papiersubstrat und einen auf dem Papiersubstrat beschichteten Film aufweist, wobei der Film oxidierte Stärke in einer Menge von 5-95 %, beispielsweise 10-50 %, beispielsweise 10-30 %, bezogen auf das Trockengewicht des Films, und Dextrin, beispielsweise Maltodextrin, in einer Menge von 95-5 %, beispielsweise 90-50 %, beispielsweise 90-70 %, bezogen auf das Trockengewicht des Films, aufweist;
b) Befeuchten eines Abschnitts des Films; und
c) Heißversiegeln des befeuchteten Abschnitts.

2. Verfahren nach Anspruch 1, wobei das Papierprodukt in Schritt c) mit sich selbst versiegelt wird.

3. Verfahren nach Anspruch 1, wobei das Papierprodukt in Schritt c) mit einem zweiten Papierprodukt versiegelt wird.

4. Verfahren nach Anspruch 3, wobei das zweite Papierprodukt ein zweites Papiersubstrat und einen auf dem zweiten Papiersubstrat beschichteten zweiten Film aufweist, wobei der zweite Film oxidierte Stärke und Dextrin, beispielsweise Maltodextrin, aufweist.

5. Verfahren nach einem der Ansprüche 1-4, wobei der Film ferner ein Nassfestmittel aufweist.

6. Verfahren nach Anspruch 5, wobei das Nassfestmittel in einer Menge von 3-7 %, beispielsweise 4-6 %, bezogen auf das Trockengewicht des Films, vorliegt.

7. Verfahren nach einem der vorhergehenden Ansprüche, wobei die Grammatur des Films 0,5-5 g/m² beträgt, beispielsweise 0,5-3 g/m², beispielsweise 1-3 g/m².

8. Verfahren nach einem der vorhergehenden Ansprüche, wobei der Film mindestens 50 %, beispielsweise mindestens 70 %, beispielsweise mindestens 90 %, oxidierte Stärke und Dextrin, bezogen auf das Trockengewicht des Films, aufweist.

9. Verfahren nach einem der vorhergehenden Ansprüche, wobei das Papiersubstrat ein maschinenglattes (MG) Papier mit einer glatten Seite und einer rauen Seite ist und der Film auf der glatten Seite oder auf der rauen Seite des Papiersubstrats beschichtet ist.

10. Verfahren nach einem der vorhergehenden Ansprüche, wobei das Verfahren ein Verfahren zur Herstellung eines Papierstab ist, das ferner den folgenden Schritt aufweist:
b1) Aufrollen des Papiers zu einem Papierstab, wobei Schritt b1) nach Schritt a) und vor Schritt c) durchgeführt wird.

11. Papierstab, hergestellt nach Anspruch 10.

12. Wattestäbchen, das den nach Anspruch 10 hergestellten Papierstab aufweist.

13. Versiegelte Verpackung oder Behälter, aufweisend ein Papierprodukt, das ein Papiersubstrat und einen auf dem Papiersubstrat beschichteten Film aufweist, wobei der Film oxidierte Stärke und Dextrin, beispielsweise Maltodextrin, aufweist.

14. Verpackung oder Behälter nach Anspruch 13, wobei die Verpackung oder der Behälter eine Siegelnaht und ein in der Verpackung oder dem Behälter angeordnetes Produkt aufweist, wobei die Siegelnaht so angeordnet ist, dass das Produkt innerhalb der Verpackung oder des Behälters versiegelt ist.

15. Verpackung oder Behälter nach Anspruch 13, wobei die Verpackung oder der Behälter eine Siegelung aufweist, die so angeordnet ist, dass die Verpackung oder der Behälter teilweise geöffnet ist, so dass ein Produkt eingeführt und entnommen werden kann, ohne die Siegelnaht oder die Verpackung oder den Behälter zu beschädigen.

## Revendications

1. Procédé de fabrication d'un emballage, d'un récipient ou d'un bâtonnet en papier, comprenant les étapes suivantes :
a) la fourniture d'un produit en papier comprenant un substrat en papier et un film enduit sur le substrat en papier, dans lequel le film comprend de l'amidon oxydé en une quantité comprise entre 5 et 95 %, telle que comprise entre 10 et 50 %, telle que comprise entre 10 et 30 %, sur la base du poids sec du film, et de la dextrine, telle que la maltodextrine, en une quantité comprise entre 95 et 5 %, telle que comprise entre 90 et 50 %, telle que comprise entre 90 et 70 %, sur la base du poids sec du film ;
b) le mouillage d'une partie du film ; et
c) le thermoscellage de la partie mouillée.

2. Procédé selon la revendication 1, dans lequel le produit en papier est scellé sur lui-même à l'étape c).

3. Procédé selon la revendication 1, dans lequel le produit en papier est scellé sur un deuxième produit en papier à l'étape c).

4. Procédé selon la revendication 3, dans lequel le deuxième produit en papier comprend un deuxième substrat en papier et un deuxième film enduit sur le deuxième substrat en papier, dans lequel le deuxième film comprend de l'amidon oxydé et de la dextrine, telle que la maltodextrine.

5. Procédé selon l'une quelconque des revendications 1 à 4, dans lequel le film comprend en outre un agent de résistance à l'état humide.

6. Procédé selon la revendication 5, dans lequel l'agent de résistance à l'état humide est présent en une quantité comprise entre 3 et 7 %, telle que comprise entre 4 et 6 %, par rapport au poids sec du film.

7. Procédé selon l'une quelconque des revendications précédentes, dans lequel le grammage du film est compris entre 0,5 et 5 g/m², tel que compris entre 0,5 et 3 g/m², tel que compris entre 1 et 3 g/m².

8. Procédé selon l'une quelconque des revendications précédentes, dans lequel le film comprend au moins 50 %, tel qu'au moins 70 %, tel qu'au moins 90 % d'amidon oxydé et de dextrine par rapport au poids sec du film.

9. Procédé selon l'une quelconque des revendications précédentes, dans lequel le substrat en papier est un papier glacé à la machine (MG) ayant un côté lisse et un côté rugueux, et le film est appliqué sur le côté lisse ou le côté rugueux du substrat en papier.

10. Procédé selon l'une quelconque des revendications précédentes, dans lequel le procédé est un procédé de formation d'un bâtonnet en papier comprenant en outre l'étape :
b1) de laminage du papier en un bâtonnet en papier, dans lequel l'étape b1) est réalisée après l'étape a) et avant l'étape c).

11. Bâtonnet en papier produit selon la revendication 10.

12. Coton-tige comprenant le bâtonnet en papier produit selon la revendication 10.

13. Emballage ou conteneur scellé comprenant un produit en papier comprenant un substrat en papier et un film enduit sur le substrat en papier, dans lequel le film comprend de l'amidon oxydé et de la dextrine, telle que la maltodextrine.

14. Emballage ou récipient selon la revendication 13, dans lequel l'emballage ou le récipient comprend une ligne de scellage et un produit à l'intérieur de l'emballage ou du récipient, dans lequel la ligne de scellage est agencée de telle sorte qu'un produit est scellé à l'intérieur de l'emballage ou du récipient.

15. Emballage ou récipient selon la revendication 13, dans lequel l'emballage ou le récipient comprend un scellage agencé de telle sorte que l'emballage ou le récipient soit partiellement ouvert afin qu'un produit puisse être inséré et retiré sans rompre la ligne de scellage ou l'emballage ou le récipient.
